# EUROPEAN PATENT SPECIFICATION

(11) **EP 3 845 230 B1**
(45) Date of publication and mention of the grant of the patent: **12.07.2023**
(21) Application number: 19855123.6
(22) Date of filing: 30.08.2019
(51) Int. Cl.: A61K 31/519, A61K 9/14, A61K 9/20, A61K 9/48, A61K 47/32, A61K 47/38, A61P 21/02, A61P 25/04, A61P 25/08, A61P 25/18, A61P 25/28, A61P 29/02, A61P 43/00

(54) **PHARMACEUTICAL COMPOSITION FOR ORAL ADMINISTRATION**
PHARMAZEUTISCHE ZUSAMMENSETZUNG ZUR ORALEN VERABREICHUNG
COMPOSITION PHARMACEUTIQUE POUR ADMINISTRATION PAR VOIE ORALE

(30) Priority: 31.08.2018 JP 2018163234
(43) Date of publication of application: 07.07.2021
(73) Proprietor: Astellas Pharma Inc., Chuo-ku Tokyo 103-8411 (JP)
(72) Inventor: KOJIMA Ryo, Tokyo 103-8411 (JP); SAKAI Toshiro, Tokyo 103-8411 (JP); AZUMA Ryota, Tokyo 103-8411 (JP); TANAKA Marina, Tokyo 103-8411 (JP)
(74) Representative: Bates, Philip Ian
(86) International application number: PCT/JP2019/034051
(87) International publication number: WO 2020/045607

(56) References cited:
- WO-A1-2015/056771
- JP-A- 2016 538 256
- US-A1- 2015 111 876
- US-A9- 2016 152 632
- WALZER MARK ET AL: "Single- and Multiple-Dose Safety, Tolerability, and Pharmacokinetic Profiles of ASP8062: Results From 2 Phase 1 Studies", CLINICAL PHARMACOLOGY IN DRUG DEVELOPMENT, vol. 9, no. 3, 1 April 2020 (2020-04-01), pages 297-306, XP055913063, GB ISSN: 2160-763X, DOI: 10.1002/cpdd.766 Retrieved from the Internet: URL:https://onlinelibrary.wiley.com/doi/fu ll-xml/10.1002/cpdd.766>
- TAGAWA MAYA et al.: "Effective of various Disintegrants on Drug Release Behavior from Tabl", Journal of Pharmaceutical Science and Technology, Japan, vol. 63, no. 4, 11 August 2003 (2003-08-11), pages 238-248, XP003019852,

## Description

### TECHNICAL FIELD

The present invention relates to a stable pharmaceutical composition for oral administration with rapid drug dissolution properties comprising 6-(4,4-dimethylcyclohexyl)-4-[(1,1-dioxo-1λ⁶-thiomorpholin-4-yl)methyl]-2-methylthieno[2,3-d]pyrimidine or a pharmaceutically acceptable salt thereof.

More particularly, the present invention relates to a pharmaceutical composition for oral administration comprising 6-(4,4-dimethylcyclohexyl)-4-[(1,1-dioxo-1λ⁶-thiomorpholin-4-yl)methyl]-2-methylthieno[2,3-d]pyrimidine or a pharmaceutically acceptable salt thereof, and a water-swellable substance.

### BACKGROUND ART

6-(4,4-Dimethylcyclohexyl)-4- [(1,1-dioxo-1^{λ6}-thiomorpholin-4-yl)methyl]-2-methylthieno[2,3-d]pyrimidine (hereinafter sometimes referred to as compound A. "1,1-dioxo-1λ⁶-thiomorpholin-4-yl" is also called "1,1-dioxidethiomorpholine-4-yl".) is a compound represented by the following chemical structural formula. A pharmaceutical composition containing compound A or a pharmaceutically acceptable salt thereof has been reported to be useful, as a GABA_{B} positive allosteric modulator, as an agent for preventing and/or treating, for example, schizophrenia, cognitive impairment associated with schizophrenia (CIAS), cognitive impairment, fragile X syndrome, autism spectrum disorder, spasticity, anxiety disorder, substance addiction, pain, fibromyalgia, Charcot-Marie-Tooth disease, or the like (Patent literature 1, or the equivalent US 2015/111876 A1). However, a stable pharmaceutical composition for oral administration with rapid drug dissolution properties comprising compound A or a pharmaceutically acceptable salt thereof is not disclosed. 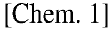 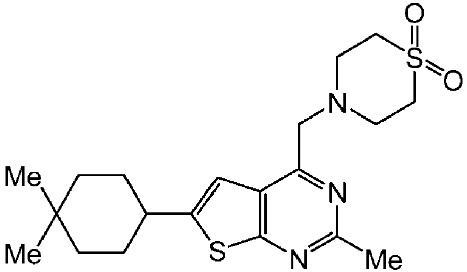 (wherein Me is a methyl group.)

When compound A was formulated, the solubility of compound A was confirmed based on a Dissolution Test of the Japanese Pharmacopoeia, Seventeenth Edition, and there was a large difference between an acidic pH range and a neutral pH range. That is to say, the solubility of compound A in an acidic pH range (first fluid for a Dissolution Test of the Japanese Pharmacopoeia, Seventeenth Edition) was approximately 41 µg/mL, whereas the solubility thereof at a neutral pH range (second fluid for a Dissolution Test of the Japanese Pharmacopoeia, Seventeenth Edition) was approximately 1.2 µg/mL, and there was a difference of approximately 30 times.

When a drug is formulated, it is desirable to exhibit rapid drug dissolution properties in order to achieve the desired pharmacological effect. Further, from the viewpoint of patient safety, it is desirable that the preparation be stable during production and storage. That is to say, it is desirable that the generation of related substances be suppressed during the production and storage of the preparation, and it is desirable that the change in drug dissolution properties be small before and after storage of the preparation. This is because if the drug dissolution from the preparation is delayed when the preparation is taken, the amount of drug absorbed from the gastrointestinal mucosa is inferior, and the problem arises that the effectiveness and fast-acting properties are affected.

In order to provide a preparation with rapid drug dissolution properties, the drug must be dissolved rapidly in the stomach. Patent literature 2 discloses a pharmaceutical composition to which an acidic substance is added, because when a basic drug cinnarizine is orally administered, drug solubility is affected by fluctuations in individual gastric pH values, and also discloses that drug solubility stabilizes even when gastric pH fluctuates.

### CITATION LIST

### PATENT LITERATURE

[Patent literature 1] WO 2015/056771
[Patent literature 2] Japanese Unexamined Patent Publication (Kokai) No. 58-134033 US 2016/152632 A9 discloses thieno-pyrimidine derivatives.
Walzer Mark ET AL: "Single- and Multiple-Dose Safety, Tolerability, and Pharmacokinetic Profiles of ASP8062: Results From 2 Phase 1 Studies",Clinical Pharmacology in Drug Development, vol.
9, no. 3, 1 April 2020, ISSN: 2160-763X, DOI: 10.1002/cpdd.766 , shows that present claimed compound A is also known as ASP8062.

### SUMMARY OF INVENTION

### TECHNICAL PROBLEM

In order to achieve a rapid dissolution of compound A or a pharmaceutically acceptable salt thereof, when anhydrous citric acid, which was an acidic substance, was placed in the vicinity of compound A, it showed rapid drug dissolution properties. However, when the storage stability of compound A in a physical mixture of anhydrous citric acid and compound A was confirmed, the total amount of related substances of compound A increased remarkably (Experimental Example 1), and thus it was difficult to use anhydrous citric acid. Further, in addition to anhydrous citric acid, it was found that compound A chemically reacted with many pharmaceutical additives. Therefore, in developing a pharmaceutical composition for oral administration containing compound A or a pharmaceutically acceptable salt thereof, it is necessary to consider the stability of compound A in addition to achieving rapid dissolution properties.

An object of the present invention is, in a pharmaceutical composition for oral administration comprising compound A or a pharmaceutically acceptable salt thereof, to provide a stable pharmaceutical composition for oral administration with rapid drug dissolution properties.

### SOLUTION TO PROBLEM

Under these circumstances, the present inventors focused on, in particular, the dissolution properties of compound A and the stability of compound A, and conducted intensive studies. As a result, the present inventors found that pharmaceutical compositions for oral administration comprising a water-swellable substance(s) maintained rapid drug dissolution properties and exhibited a good stability of compound A, and completed the present invention.

The present invention relates to:
[1] a pharmaceutical composition for oral administration comprising 6-(4,4-dimethylcyclohexyl)-4-[(1,1-dioxo-1λ⁶-thiomorpholin-4-yl)methyl]-2-methylthieno[2,3-d]pyrimidine or a pharmaceutically acceptable salt thereof, and a water-swellable substance, wherein the water-swellable substance is one, or two or more selected from the group consisting of a polymer compound obtained by condensation polymerization of β-glucose, a polymer compound obtained by condensation polymerization of α-glucose, and a polymer compound having a pyrrolidone functional group;
[2] the pharmaceutical composition for oral administration of [1], wherein the water-swellable substance is one, or two or more selected from the group consisting of:
   i) carmellose, carmellose calcium, croscarmellose sodium, and low substituted hydroxypropylcellulose, which are the polymer compounds obtained by condensation polymerization of β-glucose,
   ii) corn starch, potato starch, rice starch, partially pregelatinized starch, and pregelatinized starch, which are the polymer compounds obtained by condensation polymerization of α-glucose, and
   iii) crospovidone, which is the polymer compound having a pyrrolidone functional group;
[3] the pharmaceutical composition for oral administration of [2], wherein the water-swellable substance is the polymer compound obtained by condensation polymerization of β-glucose, and the polymer compound obtained by condensation polymerization of β-glucose is one, or two or more selected from the group consisting of carmellose, carmellose calcium, croscarmellose sodium, and low substituted hydroxypropylcellulose;
[4] the pharmaceutical composition for oral administration of [3], wherein the polymer compound obtained by condensation polymerization of β-glucose is low substituted hydroxypropylcellulose;
[5] the pharmaceutical composition for oral administration of [2], wherein the water-swellable substance is the polymer compound obtained by condensation polymerization of α-glucose, and the polymer compound obtained by condensation polymerization of α-glucose is one, or two or more selected from the group consisting of corn starch, potato starch, rice starch, partially pregelatinized starch, and pregelatinized starch;
[6] the pharmaceutical composition for oral administration of [2], wherein the water-swellable substance is the polymer compound having a pyrrolidone functional group, and the polymer compound having a pyrrolidone functional group is crospovidone;
[7] the pharmaceutical composition for oral administration of [2], wherein the water-swellable substance is one, or two or more selected from the group consisting of low substituted hydroxypropylcellulose, corn starch, and crospovidone;
[8] the pharmaceutical composition for oral administration of any one of [1] to [7], wherein the water-swellable substance with respect to a weight of 6-(4,4-dimethylcyclohexyl)-4-[(1,1-dioxo-1λ⁶-thiomorpholin-4-yl)methyl]-2-methylthieno[2,3-d]pyrimidine or a pharmaceutically acceptable salt thereof is 20% by weight to 6000% by weight;
[9] the pharmaceutical composition for oral administration of any one of [1] to [8], wherein the pharmaceutical composition for oral administration is selected from the group consisting of a tablet, a capsule, a granule, and a powder; and
[10] use of a water-swellable substance in the manufacture of a stable pharmaceutical composition for oral administration comprising 6-(4,4-dimethylcyclohexyl)-4-[(1,1-dioxo-1λ⁶-thiomorpholin-4-yl)methyl]-2-methylthieno[2,3-d]pyrimidine or a pharmaceutically acceptable salt thereof, said water-swellable substance being one, or two or more selected from the group consisting of low substituted hydroxypropylcellulose, corn starch, and crospovidone.

### ADVANTAGEOUS EFFECTS OF INVENTION

According to the present invention, a stable pharmaceutical composition for oral administration with rapid drug dissolution properties comprising compound A or a pharmaceutically acceptable salt thereof can be provided.

### DESCRIPTION OF EMBODIMENTS

The term "stable" as used herein means that a pharmaceutical composition for oral administration has high storage stability and/or that a pharmaceutical composition for oral administration has high dissolution stability. The term "storage stability" as used herein means the stability of compound A or a pharmaceutically acceptable salt thereof contained in the pharmaceutical composition for oral administration when the pharmaceutical composition for oral administration is stored under certain conditions, and it can be evaluated by the change in the total amount of related substances derived from the decomposition or the like of compound A, or the change in the amount of a specific related substance derived therefrom. That is to say, the fact that the amount of related substances of compound A does not increase significantly means that the pharmaceutical composition for oral administration has high storage stability. On the other hand, the fact that when the pharmaceutical composition for oral administration is stored under certain storage conditions, the dissolution rate of compound A from the pharmaceutical composition for oral administration does not change significantly, in particular, it does not decrease significantly, means that the pharmaceutical composition for oral administration has high "dissolution stability".

The storage conditions for evaluating these stabilities can be appropriately set according to the purpose, for example, by changing heat, light, temperature, and/or humidity, or by changing the storage period. More particularly, for example, the pharmaceutical composition for oral administration may be placed in an unsealed container, a sealed container, an airtight container, or a closed container, and stored under desired conditions, for example, at 70°C for 9 days, at 40°C and a relative humidity of 75% (hereinafter "a relative humidity of X%" may be sometimes abbreviated as "X%RH") for 6 months, at 40°C, 75%RH for 3 months, at 40°C, 75%RH for 2 months, at 40°C, 75%RH for 1 month, at 25°C, 60%RH for 12 months, at 25°C, 60%RH for 6 months, at 25°C, 60%RH for 3 months, at 25°C, 60%RH for 1 month, under irradiation with D65 lamp (1000 Lux) specified in ISO10977 for 50 days, under irradiation with D65 lamp (1000 Lux) for 25 days, under irradiation with a xenon lamp designed to show an output similar to the D65 radiation standard for 24 hours, or the like. Both stabilities can be evaluated by measuring the amount of related substances of compound A, and the dissolution rate of compound A from the composition.

The fact that the pharmaceutical composition for oral administration "has rapid drug dissolution properties" as used herein means that, as an embodiment, when a dissolution test is carried out using 900 mL of 0.1 mol/L hydrochloric acid as a dissolution test fluid (temperature of the fluid: 37±1°C) at a paddle rotation speed of 50 rpm in a Dissolution Test, a paddle method of the Japanese Pharmacopoeia, Seventeenth Edition (hereinafter, all dissolution tests and all measurements of dissolution rate are carried out under the above conditions), the dissolution rate(s) of compound A after 30 minutes and/or 15 minutes from the beginning of the test are high.

The pharmaceutical composition for oral administration of the present invention with rapid drug dissolution properties is, as an embodiment, a pharmaceutical composition for oral administration in which the dissolution rate of compound A after 30 minutes from the beginning of the test is 75% or more, 80% or more, or 85% or more. Further, as an embodiment, it is a composition in which the dissolution rate of compound A after 15 minutes is 55% or more, 60% or more, 65% or more, 70% or more, or 75% or more. Furthermore, as an embodiment, it is a pharmaceutical composition for oral administration in which the dissolution rate of compound A is 75% or more after 30 minutes from the beginning of the test and 55% or more after 15 minutes; a pharmaceutical composition for oral administration in which the dissolution rate of compound A is 80% or more after 30 minutes from the beginning of the test and 65% or more after 15 minutes; or a pharmaceutical composition for oral administration in which the dissolution rate of compound A is 83% or more after 30 minutes from the beginning of the test and 70% or more after 15 minutes.

The pharmaceutical composition for oral administration of the present invention with high dissolution stability is, as an embodiment, a pharmaceutical composition for oral administration in which when the dissolution rates of the pharmaceutical composition for oral administration before and after storage at 70°C for 9 days are measured, the dissolution rates of compound A after 30 minutes from the beginning of the test are 75% or more, 80% or more, 83% or more, or 85% or more, both before and after storage; or a pharmaceutical composition for oral administration in which the dissolution rates of compound A after 15 minutes from the beginning of the test are 55% or more, 60% or more, 67% or more, or 75% or more, both before and after storage. Further, as an embodiment, it is a pharmaceutical composition for oral administration in which the dissolution rates of compound A, both before and after storage, are 75% or more after 30 minutes from the beginning of the test and 55% or more after 15 minutes; 80% or more after 30 minutes from the beginning of the test and 65% or more after 15 minutes; or 83% or more after 30 minutes from the beginning of the test and 70% or more after 15 minutes.

As another embodiment, when the pharmaceutical composition for oral administration is placed in a closed container, such as an aluminum bag, and the dissolution rates before and after storage at 40°C, 75%RH for 1 month are measured, it is a pharmaceutical composition for oral administration in which the dissolution rates of compound A after 30 minutes from the beginning of the test are 78% or more, 81% or more, 83% or more, or 85% or more, both before and after storage; or a pharmaceutical composition for oral administration in which the dissolution rates of compound A after 15 minutes from the beginning of the test are 65% or more, 67% or more, 71% or more, or 75% or more, both before and after storage.

Furthermore, as an embodiment, when the pharmaceutical composition for oral administration is placed in a closed container, such as an aluminum bag, and the dissolution rates before and after storage at 40°C, 75%RH for 1 month are measured, it is a pharmaceutical composition for oral administration in which the dissolution rates of compound A, both before and after storage, are 78% or more after 30 minutes from the beginning of the test and 65% or more after 15 minutes; 81% or more after 30 minutes from the beginning of the test and 67% or more after 15 minutes; or 83% or more after 30 minutes from the beginning of the test and 70% or more after 15 minutes.

The storage stability as used herein can be evaluated by measuring the change in the amount of related substances derived from compound A when the pharmaceutical composition for oral administration is stored under certain storage conditions. For example, after the pharmaceutical composition for oral administration is stored under certain conditions, the total amount of related substances of compound A contained in the pharmaceutical composition for oral administration, or a specific related substance of compound A is measured. The measurement can be carried out, for example, as shown in Experimental Example 1 described below, by measuring the peak area of compound A and the peak areas of all related substances including a related substance (hereinafter sometimes referred to as related substance A) having a relative retention time of approximately 1.8 with respect to compound A by high performance liquid chromatography (HPLC). The percentage (%) of the total peak area of related substances with respect to the sum of all peak areas including the peak area of compound A are calculated, and is compared with the value before storage to calculate the amount of change (the amount of increase)(%). Alternatively, as shown in Experimental Example 3 described below, the percentage (%) of the peak area of related substance A with respect to the sum of all peak areas obtained in the same manner is calculated, and it is also possible to evaluate the storage stability by the change over time of related substance A. For example, the storage stability of the pharmaceutical composition for oral administration can be evaluated by adopting the above various storage conditions, such as at 70°C for 9 days, at 40°C, 75%RH for 6 months, at 40°C, 75%RH for 3 months, at 40°C, 75%RH for 2 months, at 40°C, 75%RH for 1 month, or the like.

The term "high storage stability" (or also referred to as "the related substances do not increase significantly") as used herein means that the amount of increase in the total amount of related substances when the pharmaceutical composition for oral administration is stored under any of the above storage conditions is 2.0% or less. On the other hand, it is said that "the related substances increase significantly" when the amount of increase exceeds 2.0%.

The pharmaceutical composition for oral administration of the present invention with high storage stability is, as an embodiment, a pharmaceutical composition for oral administration in which the amount of increase in the total amount of related substances when stored at 40°C, 75%RH for 3 months is 1.0% or less, 0.50% or less, 0.20% or less, or 0.10% or less. Further, as another embodiment, it is a pharmaceutical composition for oral administration in which the amount of increase in the total amount of related substances when stored at 40°C, 75%RH for 3 months is 0.50% or less, 0.20% or less, or 0.10% or less.

Further, the pharmaceutical composition for oral administration of the present invention with high storage stability is, as an embodiment, a pharmaceutical composition for oral administration in which the amount of related substance A when stored at 40°C, 75%RH for 3 months is 0.20% or less, 0.15% or less, or 0.10% or less. Further, as another embodiment, it is a pharmaceutical composition for oral administration in which the amount of increase in the total amount of related substances when stored at 40°C, 75%RH for 3 months is 0.20% or less, 0.15% or less, or 0.10% or less.

Compound A or a pharmaceutically acceptable salt thereof can be easily obtained by the production method described in Patent literature 1, or by a production method similar thereto.

Among the pharmaceutically acceptable salts of compound A, compound A may form an acid addition salt with an acid. More particularly, examples thereof include acid addition salts with inorganic acids, such as hydrochloric acid, hydrobromic acid, hydroiodic acid, sulfuric acid, nitric acid, phosphoric acid, and the like; and acid addition salts with organic acids, such as formic acid, acetic acid, propionic acid, oxalic acid, malonic acid, succinic acid, fumaric acid, maleic acid, lactic acid, malic acid, mandelic acid, tartaric acid, dibenzoyl tartaric acid, ditoluoyl tartaric acid, citric acid, methanesulfonic acid, ethanesulfonic acid, benzenesulfonic acid, p-toluenesulfonic acid, aspartic acid, glutamic acid, and the like.

Compound A and a pharmaceutically acceptable salt thereof is useful as an agent for preventing and/or treating schizophrenia, cognitive impairment associated with schizophrenia (CIAS), cognitive impairment, fragile X syndrome, autism spectrum disorder, spasticity, anxiety disorder, substance addiction, pain, fibromyalgia, Charcot-Marie-Tooth disease, or the like.

The dose of compound A or a pharmaceutically acceptable salt thereof can be appropriately determined for each individual case in consideration of, for example, the symptoms of the disease, or the age, race, and gender of the subject to be administered, or the like.

The daily dose is, for example, 0.001 mg/kg to 100 mg/kg per body weight, 0.1 mg/kg to 30 mg/kg as an embodiment, and 0.1 mg/kg to 10 mg/kg as an embodiment, which is administered once or divided into two to four doses per day.

The content of compound A or a pharmaceutically acceptable salt thereof is, for example, 0.01% by weight to 50% by weight, with respect to the total weight of the pharmaceutical composition for oral administration.

The "water-swellable substance" that is used in the present invention is not particularly limited, as long as it swells when it comes in contact with water, and when a pharmaceutical composition for oral administration is prepared together with compound A or a pharmaceutically acceptable salt thereof, it does not affect the stability of compound A, achieves rapid dissolution of compound A, and imparts dissolution stability. As such water-swellable substances, one, or two or more selected from polymer compounds obtained by condensation polymerization of β-glucose, polymer compounds obtained by condensation polymerization of α-glucose, and polymer compounds having a pyrrolidone functional group, may be exemplified. As an embodiment, the water-swellable substance is one, or two or more selected from the group consisting of:
i) carmellose, carmellose calcium, croscarmellose sodium, and low substituted hydroxypropylcellulose, which are the polymer compounds obtained by condensation polymerization of β-glucose,
ii) corn starch, potato starch, rice starch, sodium starch glycolate, partially pregelatinized starch, and pregelatinized starch, which are the polymer compounds obtained by condensation polymerization of α-glucose, and
iii) crospovidone, which is the polymer compound having a pyrrolidone functional group.
As an embodiment, the water-swellable substance is one, or two or more selected from the group consisting of:
i) carmellose, carmellose calcium, croscarmellose sodium, and low substituted hydroxypropylcellulose, which are the polymer compounds obtained by condensation polymerization of β-glucose,
ii) corn starch, potato starch, rice starch, partially pregelatinized starch, and pregelatinized starch, which are the polymer compounds obtained by condensation polymerization of α-glucose, and
iii) crospovidone, which is the polymer compound having a pyrrolidone functional group. As an embodiment, the water-swellable substance is one, or two or more selected from low substituted hydroxypropylcellulose, corn starch, and crospovidone. The water-swellable substance is, as an embodiment, low substituted hydroxypropylcellulose.

These water-swellable substances can be used alone or in combination of two or more.

As another embodiment of the water-swellable substance, a polymer compound not having a carboxy group (-COOH) may be exemplified. The polymer compound not having a carboxy group is, more particularly, low substituted hydroxypropylcellulose, corn starch, potato starch, rice starch, partially pregelatinized starch, pregelatinized starch, or crospovidone. Further, as another embodiment of the water-swellable substance, a nonionic polymer compound may be exemplified. The nonionic polymer compound is, more particularly, low substituted hydroxypropylcellulose, corn starch, potato starch, rice starch, partially pregelatinized starch, pregelatinized starch, or crospovidone.

These water-swellable substances can be used alone or in combination of two or more.

As the "polymer compound obtained by condensation polymerization of β-glucose" that is used as the water-swellable substance in the present invention, for example, carmellose, carmellose calcium, croscarmellose sodium, low substituted hydroxypropylcellulose, or the like may be exemplified. The "polymer compound obtained by condensation polymerization of β-glucose" that is used as the water-swellable substance is, as an embodiment, croscarmellose sodium or low substituted hydroxypropylcellulose. Further, as an embodiment, it is low substituted hydroxypropylcellulose.

The polymer compound obtained by condensation polymerization of β-glucose can be used as the water-swellable substance alone or in combination of two or more.

The low substituted hydroxypropylcellulose is a low substituted hydroxypropyl ether of cellulose, as described in the Japanese Pharmacopoeia, Seventeenth Edition. Low substituted hydroxypropylcellulose is defined as one containing 5.0% to 16.0% of hydroxypropoxy group (-OC₃H₆OH), when dried. For example, L-HPC LH21 (manufactured by Shin-Etsu Chemical) or the like may be exemplified.

The low substituted hydroxypropylcellulose can be used alone or in combination of two or more series having different average particle sizes or 90% integrated particle sizes.

The "polymer compounds obtained by condensation polymerization of α-glucose" that is used as the water-swellable substance in the present invention is, for example, as an embodiment, corn starch, potato starch, rice starch, sodium starch glycolate, partially pregelatinized starch, or pregelatinized starch. Further, as an embodiment, it is corn starch, potato starch, rice starch, partially pregelatinized starch, or pregelatinized starch. Further, as an embodiment, it is corn starch.

The polymer compound obtained by condensation polymerization of α-glucose can be used as the water-swellable substance alone or in combination of two or more.

The "polymer compound having a pyrrolidone functional group" that is used as the water-swellable substance in the present invention is, for example, crospovidone or the like.

The water-swellable substance can be blended by an arbitrary method to prepare the pharmaceutical composition for oral administration of the present invention, as long as the desired effects described in the present specification are achieved. More particularly, for example, compound A or a pharmaceutically acceptable salt thereof may be simply mixed with the water-swellable substance, or compound A or a pharmaceutically acceptable salt thereof and the water-swellable substance may be granulated.

The content of the water-swellable substance is not particularly limited, as long as the pharmaceutical composition for oral administration containing compound A or a pharmaceutically acceptable salt thereof is stable and exhibits rapid drug dissolution properties. The content ratio of the water-swellable substance to the weight of compound A or a pharmaceutically acceptable salt thereof is, for example, 20% by weight to 10000% by weight, 20% by weight to 6000% by weight as an embodiment, 50% by weight to 3000% by weight as an embodiment, 70% by weight to 1000% by weight as an embodiment, 100% by weight to 700% by weight as an embodiment, 100% by weight to 200% by weight as an embodiment, and 130% by weight to 200% by weight as an embodiment. The content ratio of the water-swellable substance to the total weight of the pharmaceutical composition for oral administration is 1% by weight to 80% by weight as an embodiment, 5% by weight to 50% by weight as an embodiment, 10% by weight to 45% by weight as an embodiment, and 10% by weight to 30% by weight as an embodiment. The upper limit and the lower limit of the content ratio of the water-swellable substance can be arbitrarily combined as desired.

In the pharmaceutical composition for oral administration, the state of the water-swellable substance is, as an embodiment, a uniformly dispersed state, and as an embodiment, a uniformly present state. Since the water-swellable substance is uniformly dispersed or uniformly present in the pharmaceutical composition for oral administration, water easily penetrates into the pharmaceutical composition for oral administration, and rapid drug dissolution properties can be imparted to the pharmaceutical composition for oral administration.

The pharmaceutical composition for oral administration of the present invention may be, for example, a tablet, a capsule, a granule, a powder, or the like, and is preferably a tablet.

The pharmaceutical composition for oral administration of the present invention is formulated by appropriately using various pharmaceutical additives as desired, as long as the desired effects described in the present specification are achieved. The pharmaceutical additives are not particularly limited as long as it is pharmaceutically acceptable and pharmacologically acceptable. For example, an excipient, a binder, an acidulant, a foaming agent, a sweetener, a flavor, a lubricant, a colorant, an antioxidant, a surfactant, a fluidizer, or the like, can be used.

Examples of the excipient include sugar alcohols, such as D-mannitol, D-sorbitol, erythritol, xylitol, and the like; sugars, such as starch, lactose, sucrose, dextran (for example, dextran 40), glucose, and the like; and others, such as gum arabic, pullulan, light anhydrous silicic acid, synthetic aluminum silicate, magnesium aluminometasilicate, and the like.

Examples of the binder include gum arabic, hypromellose, hydroxypropylcellulose, hydroxyethylcellulose, and the like.

Examples of the acidulant include tartaric acid, malic acid, and the like.

Examples of the foaming agent include sodium bicarbonate and the like.

Examples of the sweetener include sodium saccharin, dipotassium glycyrrhizinate, aspartame, stevia, thaumatin, and the like.

Examples of the flavor include lemon, orange, menthol, and the like.

Examples of the lubricant include magnesium stearate, calcium stearate, sodium stearyl fumarate, talc, and the like.

Examples of the colorant include yellow ferric oxide, red ferric oxide, black iron oxide, and the like.

Examples of the antioxidant include ascorbic acid, tocopherol, dibutylhydroxytoluene, and the like.

Examples of the surfactant include polysorbate 80, polyoxyethylene hydrogenated castor oil, and the like.

Examples of the fluidizer include light anhydrous silicic acid and the like.

These pharmaceutical additives can be added alone or in combination of two or more in appropriate amounts.

The weight of the pharmaceutical composition for oral administration of the present invention is not particularly limited, but is 10 to 960 mg as an embodiment, 55 to 650 mg as an embodiment, 85 to 600 mg as an embodiment, and 110 to 300 mg as an embodiment.

The pharmaceutical composition for oral administration of the present invention can be produced by known methods comprising the steps of, for example, pulverization of compound A or a pharmaceutically acceptable salt thereof, granulation, drying, mixing, molding (tableting), and the like.

Hereinafter, a method of producing the pharmaceutical composition for oral administration of the present invention will be explained, but these do not limit the present invention.

### Pulverization step

The pulverization method is not particularly limited as long as compound A or a pharmaceutically acceptable salt thereof can be pulverized in an ordinary pharmaceutical manner. Examples of an apparatus include a hammer mill, a ball mill, a jet mill, and a pin mill, and it is a pin mill as an embodiment.

### Granulation step

The granulation method is not particularly limited as long as the pulverized product can be granulated in an ordinary pharmaceutical manner. Examples of an apparatus include a fluidized bed granulator, a high shear granulator, an extrusion granulator, a tumbling fluidized bed granulator, a dry granulator, a twin-screw extruder, and the like, and it is a fluidized bed granulator as an embodiment.

A granulated product means a product obtained by mixing at least compound A or a pharmaceutically acceptable salt thereof, and optionally excipients, and granulating with a binder. The granulated product may further contain the water-swellable substance.

### Drying step

The drying method is not particularly limited as long as the granulated product can be dried in an ordinary pharmaceutical manner. Examples of an apparatus include a forced-air dryer, a dryer under reduced pressure, a vacuum dryer, a fluidized bed dryer, and the like. If desired, after drying, the dried product may be sieved and sized using a sieve, a comil, or the like.

### Mixing step

The mixing method is not particularly limited as long as each component can be uniformly mixed in an ordinary pharmaceutical manner. Examples of a mixing method not using an apparatus include bag mixing by shaking using a plastic bag, and stirring mixing using a mortar and pestle. Examples of an apparatus include a V-type mixer, a ribbon-type mixer, a container mixer, a high-speed stirring mixer, and the like. The mixing conditions are not particularly limited as long as they are appropriately selected.

### Molding (tableting) step

The molding method is not particularly limited as long as the mixed product can be molded in an ordinary pharmaceutical manner. Examples of an apparatus include a rotary tableting machine, a single punch tableting machine, an oil press, and the like.

In the molding step, for example, a tableting method in which a granulated product containing compound A or a pharmaceutically acceptable salt thereof, or a mixed product prepared by adding and mixing various pharmaceutical additives such as a lubricant to the granulated product (a mixed product before forming, in particular, a mixed product before tableting) is molded to form tablets, may be used. In this case, the water-swellable substance is blended with the granulated product or the mixed product before forming, or a direct tableting method in which compound A or a pharmaceutically acceptable salt thereof, the water-swellable substance, and optionally appropriate pharmaceutical additives are mixed, and molded to form tablets, may also be used.

The present invention includes use of a water-swellable substance in the manufacture of a stable pharmaceutical composition for oral administration comprising compound A or a pharmaceutically acceptable salt thereof, in particular, the water-swellable substance being one, or two or more selected from the group consisting of low substituted hydroxypropylcellulose, corn starch, and crospovidone.

With respect to the terms "pharmaceutical composition for oral administration comprising compound A or a pharmaceutically acceptable salt thereof, "water-swellable substance", "low substituted hydroxypropylcellulose", "corn starch", "crospovidone" and the like, which are used in the "use" of the present invention, the explanations therefor described in the pharmaceutical composition for oral administration of the present invention can be directly applied.

According to the "use" of the present invention, in providing the pharmaceutical composition for oral administration comprising compound A or a pharmaceutically acceptable salt thereof, high stability and rapid drug dissolution properties can be exhibited.

With respect to the content, the blending method, and the like of each component in the "use" of the present invention, the explanations therefor described in the pharmaceutical composition for oral administration of the present invention can be directly applied.

The present invention includes a method for stabilizing a pharmaceutical composition for oral administration comprising compound A or a pharmaceutically acceptable salt thereof, by using a water-swellable substance in the pharmaceutical composition for oral administration, in particular, the water-swellable substance being one, or two or more selected from the group consisting of low substituted hydroxypropylcellulose, corn starch, and crospovidone.

With respect to the terms "pharmaceutical composition for oral administration comprising compound A or a pharmaceutically acceptable salt thereof, "water-swellable substance", "low substituted hydroxypropylcellulose", "corn starch", "crospovidone" and the like, which are used in the "stabilization" of the present invention, the explanations therefor described in the pharmaceutical composition for oral administration of the present invention can be directly applied.

The term "stabilization" as used herein means the stabilization of compound A or a pharmaceutically acceptable salt thereof in the pharmaceutical composition for oral administration, and/or the stabilizing effect of dissolution properties of the pharmaceutical composition for oral administration.

With respect to the content, the blending method, and the like of each component in the "stabilization" of the present invention, the explanations therefor described in the pharmaceutical composition for oral administration of the present invention can be directly applied.

### EXAMPLES

Compound A that had been prepared in accordance with the production method described in WO 2015/056771 was used.

The present invention will now be further illustrated by, but is by no means limited to, the following Examples.

In the following Examples,
Dilactose S (manufactured by Freund Corporation) as lactose hydrate,
PEARLITOL 50C (manufactured by ROQUETTE) as D-mannitol,
HPC-L (manufactured by Nippon Soda) as hydroxypropyl cellulose (HPC),
L-HPC LH-21 (manufactured by Shin-Etsu Chemical) as low substituted hydroxypropylcellulose (L-HPC),
KICCOLATE ND-2HS (manufactured by Nichirin Chemical Industries) as croscarmellose sodium,
Japanese Pharmacopoeia anhydrous citric acid (manufactured by Komatsuya Corporation) as anhydrous citric acid,
SYLYSIA 320TP (manufactured by Fuji Silysia Chemical) as light anhydrous silicic acid,
PRUV (manufactured by JRS Pharma) as sodium stearyl fumarate,
Parteck LUB MST (manufactured by Merck KGaA) as magnesium stearate,
Ceolus UF-711 (manufactured by Asahi Kasei) or Ceolus PH-102(manufactured by Asahi Kasei) as microcrystalline cellulose,
GLYCOLYS (manufactured by ROQUETTE) as sodium starch glycolate,
Polyplasdone XL (manufactured by Ashland) as crospovidone,
Japanese Pharmacopoeia Corn Starch White JPCS-W (manufactured by Japan Corn Starch) as corn starch, and
Starch 1500G (manufactured by Colorcon) as partially pregelatinized starch, were used.

### «Example 1»

A physical mixture was prepared by placing 1 part by weight of compound A and 99 parts by weight of L-HPC in a glass bottle, covering the bottle, and shaking it strongly by hand.

### <<Comparative Example 1»

A physical mixture was prepared by placing 1 part by weight of compound A and 99 parts by weight of anhydrous citric acid in a glass bottle, covering the bottle, and shaking it strongly by hand.

### «Experimental Example 1»

The physical mixtures obtained in Example 1 and Comparative Example 1 were placed in glass bottles, covered, and stored at 40°C, 75%RH for 1 month, and the total amount of related substances before and after storage was measured by the method described below. The difference in the total amount of related substances before and after storage was calculated as the amount of increase due to storage of the total amount of related substances, and the storage stability was evaluated. For comparison, compound A alone (indicated as compound A in the table) was stored under the same conditions as above, and the amount of increase in total related substances was calculated. The total amount of related substances was measured by the HPLC method under the following conditions. The total amount of related substances (%) was calculated by dividing the sum of the peak areas of each related substance by the total peak area of compound A and all related substances including related substance A. The amount of increase in total related substances (%) was calculated by subtracting the total amount of related substances before storage (%) from the total amount of related substances after storage (%). The results are shown in Table 1.

### HPLC method

▪ Measurement wavelength: 214 nm
▪ Column: YMC-Triart C8 (4.6mm×150mm, 3µm)
▪ Column temperature: a constant temperature around 40°C
▪ Mobile phase: A phosphate buffer and a mixed solution of acetonitrile and 2-propanol
▪ Flow rate: approximately 1.2 mL/min
▪ Injection amount: 10 µL

**[Table 1]**

| Increased amount of total related substances (%) (n=2) | 1 month |
|---|---|
| Compound A | 0.00 |
| Example 1 | 0.06 |
| Comparative Example 1 | 2.63 |

As shown in the above results, the increase in total related substances in Example 1 was 0.06%, but the increase in total related substances in Comparative Example 1 was 2.63%, and the related substances increased significantly. For comparison, the total related substances did not increase in compound A alone.

Furthermore, an increase in related substances was also observed in a mixture of compound A and sodium lauryl sulfate or a mixture of compound A and calcium hydrogen phosphate.

### «Example 2»

According to the formulation of Table 2, 1.00 part by weight of pulverized compound A, 69.20 parts by weight of lactose hydrate, 27.00 parts by weight of microcrystalline cellulose (UF-711), 27.00 parts by weight of L-HPC, 2.70 parts by weight of light anhydrous silicic acid, 6.75 parts by weight of sodium stearyl fumarate, and 1.35 parts by weight of magnesium stearate were mixed using a mixer to obtain a mixed product before tableting. The obtained mixed product before tableting was formed into tablets, using a tableting machine, to obtain round tablets with a weight of 135.00 mg and a diameter of 7 mm.

### «Example 3»

According to the formulation of Table 2, 25.00 parts by weight of pulverized compound A, 326.00 parts by weight of lactose hydrate, 135.00 parts by weight of microcrystalline cellulose (UF-711), 135.00 parts by weight of L-HPC, 13.50 parts by weight of light anhydrous silicic acid, 33.75 parts by weight of sodium stearyl fumarate, and 6.75 parts by weight of magnesium stearate were mixed using a mixer to obtain a mixed product before tableting. The obtained mixed product before tableting was formed into tablets, using a tableting machine, to obtain oval tablets with a weight of 675.00 mg and a major axis of 16 mm × a minor axis of 8 mm.

### «Example 4»

According to the formulation of Table 2, 30.00 parts by weight of pulverized compound A, 150.90 parts by weight of D-mannitol, 27.00 parts by weight of microcrystalline cellulose (PH-102), 54.00 parts by weight of L-HPC, 5.40 parts by weight of light anhydrous silicic acid, and 2.70 parts by weight of magnesium stearate were mixed using a mortar and pestle to obtain a mixed product before tableting. The obtained mixed product before tableting was formed into tablets, using a tableting machine, to obtain round tablets with a weight of 270.00 mg and a diameter of 9 mm.

**[Table 2]**

| (unit: part by weight) | Ex. 2 | Ex. 3 | Ex. 4 |
|---|---|---|---|
| Compound A | 1.00 | 25.00 | 30.00 |
| Lactose hydrate | 69.20 | 326.00 | - |
| D-mannitol | - | - | 150.90 |
| Microcrystalline cellulose | 27.00 | 135.00 | 27.00 |
| L-HPC | 27.00 | 135.00 | 54.00 |
| Light anhydrous silicic acid | 2.70 | 13.50 | 5.40 |
| Sodium stearyl fumarate | 6.75 | 33.75 | - |
| Magnesium stearate | 1.35 | 6.75 | 2.70 |
| Total | 135.00 | 675.00 | 270.00 |

### «Example 5»

Tablet forming was carried out according to the formulation of table 3. First, 30.00 parts by weight of pulverized compound A and 148.20 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 186.30 parts by weight of the obtained granulated product, 27.00 parts by weight of microcrystalline cellulose (PH-102), 54.00 parts by weight of L-HPC, and 2.70 parts by weight of magnesium stearate were added, and mixed using a plastic bag to obtain a mixed product before tableting. The obtained mixed product before tableting was formed into tablets, using a tableting machine, to obtain round tablets with a weight of 270.00 mg and a diameter of 9 mm.

### «Example 6»

Tablet forming was carried out according to the formulation of table 3. First, 5.00 parts by weight of pulverized compound A and 90.85 parts by weight of D-mannitol were mixed using a fluidized bed granulator to obtain a mixed product. A binder solution having a solid content of 7% by weight was prepared by dissolving 4.05 parts by weight of HPC in water. The mixed product was granulated by spraying the binder solution, and dried to obtain a granulated product.

To 99.90 parts by weight of the obtained granulated product, 13.50 parts by weight of microcrystalline cellulose (PH-102), 20.25 parts by weight of L-HPC, and 1.35 parts by weight of magnesium stearate were added, and mixed using a mixer to obtain a mixed product before tableting. The obtained mixed product before tableting was formed into tablets, using a tableting machine, to obtain round tablets with a weight of 135.00 mg and a diameter of 7 mm.

### «Example 7»

Tablet forming was carried out according to the formulation of table 3. First, 30.00 parts by weight of pulverized compound A and 161.70 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 199.80 parts by weight of the obtained granulated product, 27.00 parts by weight of microcrystalline cellulose (PH-102), 40.50 parts by weight of L-HPC, and 2.70 parts by weight of magnesium stearate were added, and mixed using a mixer to obtain a mixed product before tableting. The obtained mixed product before tableting was formed into tablets, using a tableting machine, to obtain round tablets with a weight of 270.00 mg and a diameter of 9 mm.

### «Example 8»

Tablet forming was carried out according to the formulation of table 3. First, 30.00 parts by weight of pulverized compound A and 148.20 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 186.30 parts by weight of the obtained granulated product, 27.00 parts by weight of microcrystalline cellulose (PH-102), 54.00 parts by weight of croscarmellose sodium, and 2.70 parts by weight of magnesium stearate were added, and mixed using a plastic bag to obtain a mixed product before tableting. The obtained mixed product before tableting was formed into tablets, using a tableting machine, to obtain round tablets with a weight of 270.00 mg and a diameter of 9 mm.

**[Table 3]**

| (unit: part by weight) | Ex. 5 | Ex. 6 | Ex. 7 | Ex. 8 |
|---|---|---|---|---|
| Compound A | 30.00 | 5.00 | 30.00 | 30.00 |
| D-mannitol | 148.20 | 90.85 | 161.70 | 148.20 |
| HPC | 8.10 | 4.05 | 8.10 | 8.10 |
| Microcrystalline cellulose | 27.00 | 13.50 | 27.00 | 27.00 |
| L-HPC | 54.00 | 20.25 | 40.50 | - |
| Croscarmellose sodium | - | - | - | 54.00 |
| Magnesium stearate | 2.70 | 1.35 | 2.70 | 2.70 |
| total | 270.00 | 135.00 | 270.00 | 270.00 |

### «Experimental Example 2»

A dissolution test of the tablets obtained in Examples 2 to 8 was carried out under the following conditions in accordance with a Dissolution Test, a paddle method of the Japanese Pharmacopoeia, Seventeenth Edition. The results are shown in Table 4.
▪ Paddle rotation speed: 50 rpm
▪ Test fluid: 0.1 mol/L hydrochloric acid, 900 mL
▪ Temperature of test fluid: 37±0.5°C
▪ Sampling time: 15 minutes, 30 minutes
▪ Measurement method: Ultraviolet spectroscopy (UV method (measurement wavelength: 245 nm))

**[Table 4]**

| Dissolution rate (%) | 15 minutes | 30 minutes |
|---|---|---|
| Example 2 | 87.8 | 97.1 |
| Example 3 | 75.4 | 88.0 |
| Example 4 | 82.9 | 89.5 |
| Example 5 | 83.6 | 90.1 |
| Example 6 | 97.8 | 102.7 |
| Example 7 | 79.6 | 88.1 |
| Example 8 | 83.5 | 89.6 |

As shown in the above results, the dissolution rates after 15 minutes from the beginning were 75% or more, and the dissolution rates after 30 minutes from the beginning were 85% or more, and the tablets of Examples 2 to 8 showed rapid drug dissolution properties.

### «Experimental Example 3»

The tablets obtained in Examples 6 and 7 were placed in aluminum-aluminum blisters and stored at 40°C, 75%RH for 1 month, 2 months, and 3 months, and then related substance A was measured to evaluate the storage stability. The measurement method of the related substances was the same as in Experimental Example 1, except that the injection volume was 20 µL. The amount (%) of related substance A was calculated by measuring each peak area of compound A and each related substance contained in the pharmaceutical compositions for oral administration by the HPLC method, and dividing the peak area of related substance A by the total peak area of compound A and all related substances including related substance A. The results are shown in Table 5.

**[Table 5]**

| Related substance A (%) (n=2) | 1 month | 2 months | 3 months |
|---|---|---|---|
| Example 6 | 0.09 | 0.10 | 0.08 |
| Example 7 | 0.07 | 0.08 | 0.08 |

As shown in the above results, the tablets of Examples 6 and 7 showed no increase in related substance A for 3 months, and had high storage stability.

### «Example 9»

Tablet forming was carried out according to the formulation of table 6. First, 30.00 parts by weight of pulverized compound A and 148.20 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 186.30 parts by weight of the obtained granulated product, 27.00 parts by weight of microcrystalline cellulose (PH-102), 40.50 parts by weight of L-HPC, 13.50 parts by weight of D-mannitol, and 2.70 parts by weight of magnesium stearate were added, and mixed using a plastic bag to obtain a mixed product before tableting. The obtained mixed product before tableting was formed into tablets, using a tableting machine, to obtain round tablets with a weight of 270.00 mg and a diameter of 9 mm.

### «Example 10»

Tablet forming was carried out according to the formulation of table 6. First, 30.00 parts by weight of pulverized compound A and 148.20 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 186.30 parts by weight of the obtained granulated product, 27.00 parts by weight of microcrystalline cellulose (PH-102), 27.00 parts by weight of croscarmellose sodium, 27.00 parts by weight of D-mannitol, and 2.70 parts by weight of magnesium stearate were added, and mixed using a plastic bag to obtain a mixed product before tableting. The obtained mixed product before tableting was formed into tablets, using a tableting machine, to obtain round tablets with a weight of 270.00 mg and a diameter of 9 mm.

### «Example 11»

Tablet forming was carried out according to the formulation of table 6. First, 30.00 parts by weight of compound A and 30.00 parts by weight of D-mannitol were mixed and pulverized to obtain a mixed, pulverized product. Next, 60.00 parts by weight of the mixed, pulverized product and 131.70 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 199.80 parts by weight of the obtained granulated product, 27.00 parts by weight of microcrystalline cellulose (PH-102), 40.50 parts by weight of L-HPC, and 2.70 parts by weight of magnesium stearate were added, and mixed using a plastic bag to obtain a mixed product before tableting. The obtained mixed product before tableting was formed into tablets, using a tableting machine, to obtain round tablets with a weight of 270.00 mg and a diameter of 9 mm.

### «Example 12»

Tablet forming was carried out according to the formulation of table 6. First, 30.00 parts by weight of compound A and 30.00 parts by weight of D-mannitol were mixed and pulverized to obtain a mixed, pulverized product. Next, 60.00 parts by weight of the mixed, pulverized product and 131.70 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 199.80 parts by weight of the obtained granulated product, 27.00 parts by weight of microcrystalline cellulose (PH-102), 40.50 parts by weight of croscarmellose sodium, and 2.70 parts by weight of magnesium stearate were added, and mixed using a plastic bag to obtain a mixed product before tableting. The obtained mixed product before tableting was formed into tablets, using a tableting machine, to obtain round tablets with a weight of 270.00 mg and a diameter of 9 mm.

### «Example 13»

Tablet forming was carried out according to the formulation of table 6. First, 30.00 parts by weight of compound A and 30.00 parts by weight of D-mannitol were mixed and pulverized to obtain a mixed, pulverized product. Next, 60.00 parts by weight of the mixed, pulverized product and 131.70 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 199.80 parts by weight of the obtained granulated product, 27.00 parts by weight of D-mannitol, 40.50 parts by weight of L-HPC, and 2.70 parts by weight of magnesium stearate were added, and mixed using a plastic bag to obtain a mixed product before tableting. The obtained mixed product before tableting was formed into tablets, using a tableting machine, to obtain round tablets with a weight of 270.00 mg and a diameter of 9 mm.

### «Example 14»

Tablet forming was carried out according to the formulation of table 6. First, 30.00 parts by weight of compound A and 30.00 parts by weight of D-mannitol were mixed and pulverized to obtain a mixed, pulverized product. Next, 60.00 parts by weight of the mixed, pulverized product and 131.70 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 199.80 parts by weight of the obtained granulated product, 27.00 parts by weight of D-mannitol, 40.50 parts by weight of sodium starch glycolate, and 2.70 parts by weight of magnesium stearate were added, and mixed using a plastic bag to obtain a mixed product before tableting. The obtained mixed product before tableting was formed into tablets, using a tableting machine, to obtain round tablets with a weight of 270.00 mg and a diameter of 9 mm.

### «Example 15»

Tablet forming was carried out according to the formulation of table 6. First, 30.00 parts by weight of compound A and 30.00 parts by weight of D-mannitol were mixed and pulverized to obtain a mixed, pulverized product. Next, 60.00 parts by weight of the mixed, pulverized product and 131.70 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 199.80 parts by weight of the obtained granulated product, 27.00 parts by weight of D-mannitol, 40.50 parts by weight of crospovidone, and 2.70 parts by weight of magnesium stearate were added, and mixed using a plastic bag to obtain a mixed product before tableting. The obtained mixed product before tableting was formed into tablets, using a tableting machine, to obtain round tablets with a weight of 270.00 mg and a diameter of 9 mm.

### «Example 16»

Tablet forming was carried out according to the formulation of table 6. First, 30.00 parts by weight of compound A and 30.00 parts by weight of D-mannitol were mixed and pulverized to obtain a mixed, pulverized product. Next, 60.00 parts by weight of the mixed, pulverized product and 131.70 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 199.80 parts by weight of the obtained granulated product, 27.00 parts by weight of D-mannitol, 40.50 parts by weight of corn starch, and 2.70 parts by weight of magnesium stearate were added, and mixed using a plastic bag to obtain a mixed product before tableting. The obtained mixed product before tableting was formed into tablets, using a tableting machine, to obtain round tablets with a weight of 270.00 mg and a diameter of 9 mm.

### «Example 17»

Tablet forming was carried out according to the formulation of table 6. First, 30.00 parts by weight of compound A and 30.00 parts by weight of D-mannitol were mixed and pulverized to obtain a mixed, pulverized product. Next, 60.00 parts by weight of the mixed, pulverized product and 131.70 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 199.80 parts by weight of the obtained granulated product, 27.00 parts by weight of D-mannitol, 40.50 parts by weight of partially pregelatinized starch, and 2.70 parts by weight of magnesium stearate were added, and mixed using a plastic bag to obtain a mixed product before tableting. The obtained mixed product before tableting was formed into tablets, using a tableting machine, to obtain round tablets with a weight of 270.00 mg and a diameter of 9 mm.

### <<Comparative Example 2»

Tablet forming was carried out according to the formulation of table 6. First, 30.00 parts by weight of compound A and 30.00 parts by weight of D-mannitol were mixed and pulverized to obtain a mixed, pulverized product. Next, 60.00 parts by weight of the mixed, pulverized product and 131.70 parts by weight of D-mannitol were mixed using a fluidized bed granulator to obtain a mixed product. A binder solution having a solid content of 7% by weight was prepared by dissolving 8.10 parts by weight of HPC in water. The mixed product was granulated by spraying the binder solution, and dried to obtain a granulated product.

To 199.80 parts by weight of the obtained granulated product, 67.50 parts by weight of D-mannitol, and 2.70 parts by weight of magnesium stearate were added, and mixed using a plastic bag to obtain a mixed product before tableting. The obtained mixed product before tableting was formed into tablets, using a tableting machine, to obtain round tablets with a weight of 270.00 mg and a diameter of 9 mm.

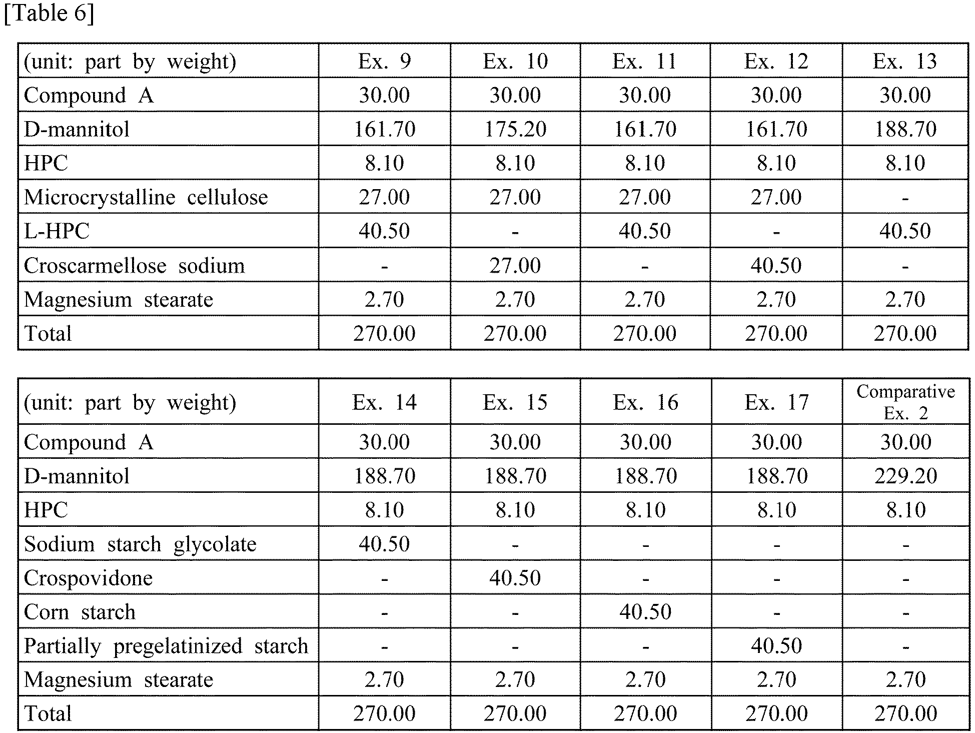

### «Experimental Example 4»

The tablets obtained in Examples 9 to 17 and Comparative Example 2 were placed in aluminum bags and stored at 70°C for 9 days or at 40°C, 75%RH for 1 month. The dissolution rates before and after storage were measured to evaluate the dissolution stability. The results are shown in Table 7.

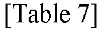

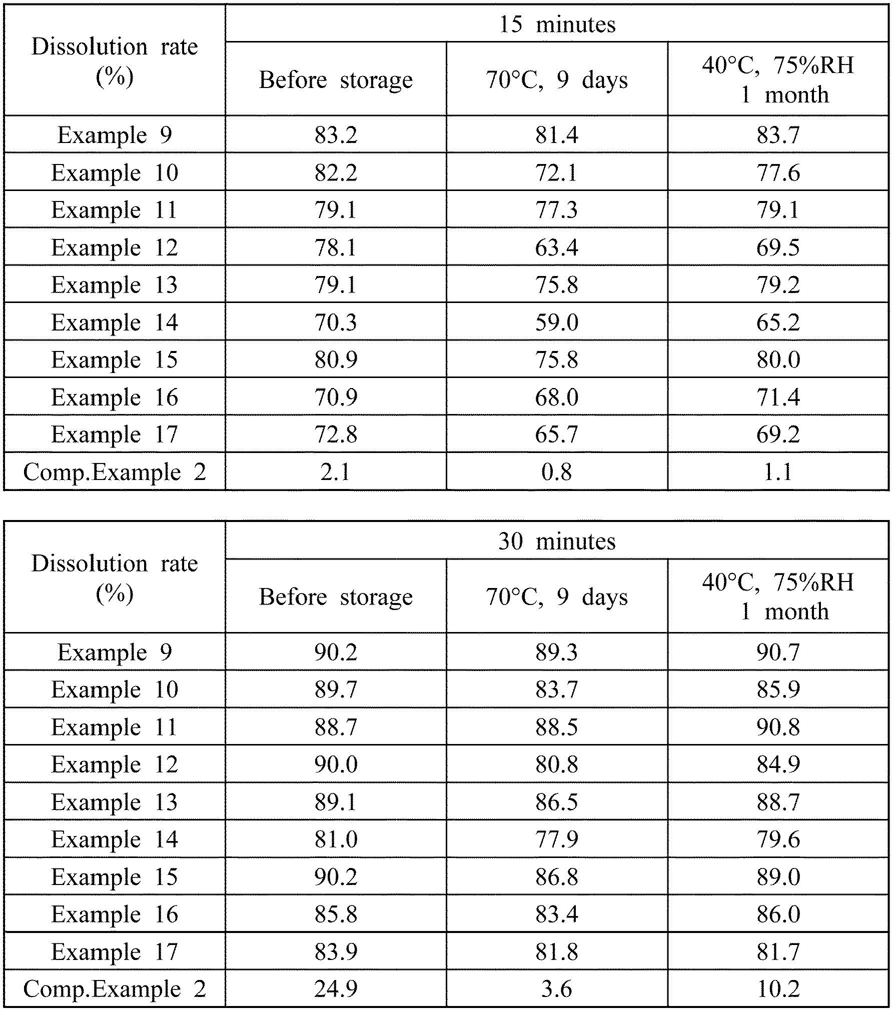

As shown in the above results, the dissolution rates of the tablets of Examples 9 to 17 stored at 70°C for 9 days were 59% or more after 15 minutes from the beginning, and 77% or more after 30 minutes from the beginning, both before and after storage, and rapid drug dissolution properties were maintained. The dissolution rates of the tablets of Examples 9 to 17 stored at 40°C, 75%RH for 1 month were 65% or more after 15 minutes from the beginning, and 79% or more after 30 minutes from the beginning, both before and after storage. These results showed that the pharmaceutical composition for oral administration of the present invention, which contained a water-swellable substance, had rapid drug dissolution properties and high dissolution stability, in comparison with Comparative Example 2 which did not contain the water-swellable substance.

### INDUSTRIAL APPLICABILITY

According to the present invention, a stable pharmaceutical composition for oral administration with rapid drug dissolution properties comprising 6-(4,4-dimethylcyclohexyl)-4-[(1,1-dioxo-1λ⁶-thiomorpholin-4-yl)methyl]-2-methylthieno[2,3-d]pyrimidine or a pharmaceutically acceptable salt thereof can be provided.

## Claims

1. A pharmaceutical composition for oral administration comprising 6-(4,4-dimethylcyclohexyl)-4-[(1,1-dioxo-1λ⁶-thiomorpholin-4-yl)methyl]-2-methylthieno[2,3-d]pyrimidine or a pharmaceutically acceptable salt thereof, and a water-swellable substance, wherein the water-swellable substance is one, or two or more selected from the group consisting of a polymer compound obtained by condensation polymerization of β-glucose, a polymer compound obtained by condensation polymerization of α-glucose, and a polymer compound having a pyrrolidone functional group.

2. The pharmaceutical composition for oral administration according to claim 1, wherein the water-swellable substance is one, or two or more selected from the group consisting of:
i) carmellose, carmellose calcium, croscarmellose sodium, and low substituted hydroxypropylcellulose, which are the polymer compounds obtained by condensation polymerization of β-glucose,
ii) corn starch, potato starch, rice starch, partially pregelatinized starch, and pregelatinized starch, which are the polymer compounds obtained by condensation polymerization of α-glucose, and
iii) crospovidone, which is the polymer compound having a pyrrolidone functional group.

3. The pharmaceutical composition for oral administration according to claim 2, wherein the water-swellable substance is the polymer compound obtained by condensation polymerization of β-glucose, and the polymer compound obtained by condensation polymerization of β-glucose is one, or two or more selected from the group consisting of carmellose, carmellose calcium, croscarmellose sodium, and low substituted hydroxypropylcellulose.

4. The pharmaceutical composition for oral administration according to claim 3, wherein the polymer compound obtained by condensation polymerization of β-glucose is low substituted hydroxypropylcellulose.

5. The pharmaceutical composition for oral administration according to claim 2, wherein the water-swellable substance is the polymer compound obtained by condensation polymerization of α-glucose, and the polymer compound obtained by condensation polymerization of α-glucose is one, or two or more selected from the group consisting of corn starch, potato starch, rice starch, partially pregelatinized starch, and pregelatinized starch.

6. The pharmaceutical composition for oral administration according to claim 2, wherein the water-swellable substance is the polymer compound having a pyrrolidone functional group, and the polymer compound having a pyrrolidone functional group is crospovidone.

7. The pharmaceutical composition for oral administration according to claim 2, wherein the water-swellable substance is one, or two or more selected from the group consisting of low substituted hydroxypropylcellulose, corn starch, and crospovidone.

8. The pharmaceutical composition for oral administration according to any one of claims 1 to 7, wherein the water-swellable substance with respect to a weight of 6-(4,4-dimethylcyclohexyl)-4-[(1,1-dioxo-1λ⁶-thiomorpholin-4-yl)methyl]-2-methylthieno[2,3-d]pyrimidine or a pharmaceutically acceptable salt thereof is 20% by weight to 6000% by weight.

9. The pharmaceutical composition for oral administration according to any one of claims 1 to 8, wherein the pharmaceutical composition for oral administration is selected from the group consisting of a tablet, a capsule, a granule, and a powder.

## Patentansprüche

1. Pharmazeutische Zusammensetzung zur oralen Verabreichung, die 6-(4,4-Dimethylcyclohexyl)-4-[(1,1-dioxo-1λ⁶-thiomorpholin-4-yl)methyl]-2-methylthieno[2,3-d]pyrimidin oder ein pharmazeutisch akzeptables Salz davon und eine in Wasser quellbare Substanz umfasst, wobei die in Wasser quellbare Substanz eine oder zwei oder mehrere aus der Gruppe bestehend aus einer Polymerverbindung, die durch Kondensationspolymerisation von β-Glucose erhalten wird, einer Polymerverbindung, die durch Kondensationspolymerisation von α-Glucose erhalten wird, und einer Polymerverbindung mit einer funktionellen Pyrrolidongruppe ausgewählte ist.

2. Pharmazeutische Zusammensetzung zur oralen Verabreichung nach Anspruch 1, wobei die in Wasser quellbare Substanz eine oder zwei oder mehrere aus der folgenden Gruppe ausgewählte ist, bestehend aus:
i) Carmellose, Carmellose-Calcium, Croscarmellose-Natrium und niedrig substituierter Hydroxypropylcellulose, die die durch Kondensationspolymerisation von β-Glucose erhaltenen Polymerverbindungen sind,
ii) Maisstärke, Kartoffelstärke, Reisstärke, teilweise vorgelierter Stärke und vorgelierter Stärke, die die durch Kondensationspolymerisation von α-Glucose erhaltenen Polymerverbindungen sind, und
iii) Crospovidon, das die Polymerverbindung mit einer funktionellen Pyrrolidongruppe ist.

3. Pharmazeutische Zusammensetzung zur oralen Verabreichung nach Anspruch 2, wobei die in Wasser quellbare Substanz die durch Kondensationspolymerisation von β-Glucose erhaltene Polymerverbindung ist und die durch Kondensationspolymerisation von β-Glucose erhaltene Polymerverbindung eine oder zwei oder mehrere aus der Gruppe bestehend aus Carmellose, Carmellose-Calcium, Croscarmellose-Natrium und niedrig substituierter Hydroxypropylcellulose ausgewählte ist.

4. Pharmazeutische Zusammensetzung zur oralen Verabreichung nach Anspruch 3, wobei die durch Kondensationspolymerisation von β-Glucose erhaltene Polymerverbindung niedrig substituierte Hydroxypropylcellulose ist.

5. Pharmazeutische Zusammensetzung zur oralen Verabreichung nach Anspruch 2, wobei die in Wasser quellbare Substanz die durch Kondensationspolymerisation von α-Glucose erhaltene Polymerverbindung ist und die durch Kondensationspolymerisation von α-Glucose erhaltene Polymerverbindung eine oder zwei oder mehrere aus der Gruppe bestehend aus Maisstärke, Kartoffelstärke, Reisstärke, teilweise vorgelierter Stärke und vorgelierter Stärke ausgewählte ist.

6. Pharmazeutische Zusammensetzung zur oralen Verabreichung nach Anspruch 2, wobei die in Wasser quellbare Substanz die Polymerverbindung mit einer funktionellen Pyrrolidongruppe ist und die Polymerverbindung mit einer funktionellen Pyrrolidongruppe Crospovidon ist.

7. Pharmazeutische Zusammensetzung zur oralen Verabreichung nach Anspruch 2, wobei die in Wasser quellbare Substanz eine oder zwei oder mehrere aus der Gruppe bestehend aus niedrig substituierter Hydroxypropylcellulose, Maisstärke und Crospovidon ausgewählte ist.

8. Pharmazeutische Zusammensetzung zur oralen Verabreichung nach einem der Ansprüche 1 bis 7, wobei die in Wasser quellbare Substanz bezogen auf das Gewicht von 6-(4,4-Dimethylcyclohexyl)-4-[(1,1-dioxo-1λ⁶-thiomorpholin-4-yl)methyl]-2-methylthieno[2,3-d]pyrimidin oder eines pharmazeutisch akzeptablen Salzes davon 20 Gew.-% bis 6000 Gew.-% beträgt.

9. Pharmazeutische Zusammensetzung zur oralen Verabreichung nach einem der Ansprüche 1 bis 8, wobei die pharmazeutische Zusammensetzung zur oralen Verabreichung aus der Gruppe bestehend aus einer Tablette, einer Kapsel, einem Granulat und einem Pulver ausgewählt ist.

## Revendications

1. Composition pharmaceutique pour administration orale comprenant de la 6-(4,4-diméthylcyclohexyl)-4-[(1,1-dioxo-11λ⁶-thiomorpholin-4-yl)méthyl]-2-méthylthiéno[2,3-d]pyrimidine ou un sel pharmaceutiquement acceptable de celle-ci, et une substance gonflable dans l'eau, dans laquelle la substance gonflable dans l'eau en est une, deux ou plus sélectionnées parmi le groupe composé d'un composé polymère obtenu par polymérisation par condensation de β-glucose, d'un composé polymère obtenu par polymérisation par condensation d'a-glucose et d'un composé polymère ayant un groupe fonctionnel pyrrolidone.

2. Composition pharmaceutique pour administration orale selon la revendication 1, dans laquelle la substance gonflable dans l'eau en est une, deux ou plus sélectionnées parmi le groupe composé de :
i) carmellose, carmellose calcique, croscarmellose sodique et hydroxypropylcellulose faiblement substituée, qui sont les composés polymères obtenus par polymérisation par condensation de β-glucose,
ii) amidon de maïs, amidon de pomme de terre, amidon de riz, amidon partiellement prégélatinisé et amidon prégélatinisé, qui sont les composés polymères obtenus par polymérisation par condensation d'a-glucose, et
iii) crospovidone, qui est le composé polymère ayant un groupe fonctionnel pyrrolidone.

3. Composition pharmaceutique pour administration orale selon la revendication 2, dans laquelle la substance gonflable dans l'eau est le composé polymère obtenu par polymérisation par condensation de β-glucose, et le composé polymère obtenu par polymérisation par condensation de β-glucose en est un, deux ou plus sélectionnés parmi le groupe composé de carmellose, carmellose calcique, croscarmellose sodique et hydroxypropylcellulose faiblement substituée.

4. Composition pharmaceutique pour administration orale selon la revendication 3, dans laquelle le composé polymère obtenu par polymérisation par condensation de β-glucose est de l'hydroxypropylcellulose faiblement substituée.

5. Composition pharmaceutique pour administration orale selon la revendication 2, dans laquelle la substance gonflable dans l'eau est le composé polymère obtenu par polymérisation par condensation d'a-glucose, et le composé polymère obtenu par polymérisation par condensation d'a-glucose en est un, deux ou plus sélectionnés parmi le groupe composé d'amidon de maïs, amidon de pomme de terre, amidon de riz, amidon partiellement prégélatinisé et amidon prégélatinisé.

6. Composition pharmaceutique pour administration orale selon la revendication 2, dans laquelle la substance gonflable dans l'eau est le composé polymère ayant un groupe fonctionnel pyrrolidone, et le composé polymère ayant un groupe fonctionnel pyrrolidone est de la crospovidone.

7. Composition pharmaceutique pour administration orale selon la revendication 2, dans laquelle la substance gonflable dans l'eau en est une, deux ou plus sélectionnées parmi le groupe composé d'hydroxypropylcellulose faiblement substituée, d'amidon de maïs et de crospovidone.

8. Composition pharmaceutique pour administration orale selon l'une quelconque des revendications 1 à 7, dans laquelle la substance gonflable dans l'eau par rapport à un poids de 6-(4,4-diméthylcyclohexyl)-4-[(1,1-dioxo-1λ⁶-thiomorpholin-4-yl)méthyl]-2-méthylthiéno[2,3-d]pyrimidine ou d'un sel pharmaceutiquement acceptable de celle-ci est de 20 % en poids à 6000 % en poids.

9. Composition pharmaceutique pour administration orale selon l'une quelconque des revendications 1 à 8, où la composition pharmaceutique pour administration orale est sélectionnée parmi le groupe composé d'un comprimé, d'une capsule, d'un granule et d'une poudre.
